# EUROPEAN PATENT SPECIFICATION

(11) **EP 4 042 925 B1**
(45) Date of publication and mention of the grant of the patent: **04.10.2023**
(21) Application number: 19957941.8
(22) Date of filing: 27.12.2019
(51) Int. Cl.: A61B 1/00, A61B 1/04, A61B 50/20, A61B 90/50, G02B 23/24

(54) **CAMERA HEAD ADAPTOR**
ADAPTER FÜR EINEN KAMERAKOPF
ADAPTATEUR DE TÊTE DE CAMÉRA

(43) Date of publication of application: 17.08.2022
(73) Proprietor: RIVERFIELD Inc., 1070052 Tokyo (JP)
(72) Inventor: TADANO, Kotaro, Tokyo 160-0017 (JP); YAMAMOTO, Nobuaki, Tokyo 160-0017 (JP)
(74) Representative: adares Patent- und Rechtsanwälte Reininger & Partner GmbB
(86) International application number: PCT/JP2019/051461
(87) International publication number: WO 2021/131027

(56) References cited:
- DE-C2- 19 955 041
- JP-A- H09 114 585
- JP-A- 2012 254 193
- JP-A- 2018 068 946
- JP-A- 2018 068 946
- JP-A- 2018 192 273
- US-A1- 2013 060 087
- US-A1- 2017 027 422

## Description

### [Technical Field]

The present invention relates to a camera head adaptor for holding an endoscope.

### [Background Art]

As a robot for holding an endoscope, for example, surgical assistance systems described in Patent Documents 1 and 2 have been proposed. Such a surgical assistance system is composed of a base unit and a robot mechanism, and the robot mechanism has a support column, a first robot arm that is attached to the tip of the support column so as to be rotatable, a second robot arm that is attached to the tip of the first robot arm so as to be rotatable, and a device support that is attached to the tip of the second robot arm. The device support is configured to accommodate an endoscope or other medical tool or device. Document JP 2018068946 discloses a prior art camera head holder with two side walls that act as a holding grip for the camera head.

[Patent Document 1] JP2016-93493A
[Patent Document 2] EP2246006A

### [Summary of the Invention]

### [Problems to be solved by the Invention]

In the surgical assistance systems described in Patent Documents 1 and 2, however, when an endoscope having a structure in which an insertion portion extends from a camera head is held, the insertion portion is clamped by the device support (see Fig. 1 of Patent Document 2), and therefore the electrical wirings and optical cables in the insertion portion are liable to be stressed. This stress may damage the electrical wirings, optical cables, etc. in the insertion portion, thus leading to the instability of the treatment tool's operation and the image capture, and there are concerns about problems such as deterioration in the image quality of captured images.

Moreover, the insertion portion is clamped by adjusting the distance between two clamping materials provided at the tip of the device support. This distance adjustment is performed while balancing the weight of the camera head and the insertion portion, and the operation is therefore somewhat complicated.

Accordingly, an object of the present invention is to provide a camera head adaptor that allows an endoscope to be detachably attached with a simple operation and that can prevent stress on electrical wirings, optical cables, etc. in an insertion portion due to a camera head being held. Another object of the present invention is to provide a camera head adaptor that can readily respond to the change in the size or shape of a camera head by adopting a simple configuration comprising a holding portion that holds the camera head and a connecting portion and a coupling portion that are provided at both ends of the holding portion.

### [Means for solving the Problems]

The invention is defined by the independent claim.

In the camera head adaptor of the present invention, the holding portion preferably has a release structure that can release holding of the camera head when an external force of a predetermined value or more is applied to the endoscope or the camera head adaptor.

In the camera head adaptor of the present invention, the holding portion is composed of an elastically deformable material and comprises a plurality of wall portions that elastically hold the camera head inserted in the holding portion while being in contact with the camera head, and the release structure is preferably configured such that the holding of the camera head is released by elastic deformation or plastic deformation of the plurality of wall portions when the external force of the predetermined value or more is applied to the endoscope or the camera head adaptor.

In the camera head adaptor of the present invention, the connecting portion preferably comprises an engaging portion that enables detachable attachment to the support structure.

In the camera head adaptor of the present invention, preferably, the support structure is a robot comprising a bendable arm portion and the connecting portion is detachably attached to the arm portion.

In the camera head adaptor of the present invention, preferably, the endoscope is configured such that an insertion portion, an electrical cable, and an optical cable extend from the camera head, the holding portion holds only the camera head of the endoscope, and the electrical cable and the optical cable extend from the coupling portion to outside.

In the camera head adaptor of the present invention, preferably, the camera head has a change in an outer shape when viewed along a direction in which the observation optical axis extends, and the holding portion has an inner surface shape corresponding to the outer shape of the camera head.

In the camera head adaptor of the present invention, the plurality of wall portions of the holding portion includes two side wall portions that extend along a direction of a central axis of the holding portion and face each other and the camera head is supported by the two side wall portions.

In the camera head adaptor of the present invention, the two side wall portions preferably have a facing distance that decreases in a front-side portion in the direction of the central axis toward a front and decreases in a rear-side portion toward a rear.

In the camera head adaptor of the present invention, preferably, the plurality of wall portions of the holding portion further includes a bottom wall portion that connects the two side wall portions to each other on a back side in an insertion direction of the camera head and the camera head is supported by the two side wall portions and the bottom wall portion.

In the camera head adaptor of the present invention, the front-side portion of the holding portion is preferably provided with a groove portion that allows the insertion portion extending from the camera head held by the holding portion to extend to outside without bending.

In the camera head adaptor of the present invention, preferably, an opening portion that allows the camera head to be inserted is formed between the two side wall portions, and when the camera head is inserted in the holding portion, an operation unit of the camera head is exposed from the opening portion.

In the camera head adaptor of the present invention, preferably, the coupling portion has a space that merges into a space accommodating the camera head in the holding portion and the electrical cable and the optical cable are connected to the camera head without bending.

### [Effect of the Invention]

According to the present invention, it is possible to provide a camera head adaptor that allows the endoscope to be detachably attached with a simple operation and that can prevent stress on electrical wirings, optical cables, etc. in the insertion portion due to the camera head being held. Moreover, by adopting a simple configuration comprising the holding portion that holds the camera head and the connecting portion and coupling portion that are provided at both ends of the holding portion, it is possible to provide a camera head adaptor that can readily respond to the change in the size or shape of the camera head.

### [Brief Description of Drawings]

FIG. 1(a) is a perspective view illustrating a state in which an endoscope and a holder are attached to a camera head adaptor according to one or more embodiments of the present invention, and FIG. 1(b) is a perspective view illustrating a state in which the endoscope and the holder are detached.
FIG. 2 is a perspective view illustrating a state in which the holder and the arm portion of a robot are separated from each other.
FIG. 3(a) is a plan view of the camera head adaptor as viewed from above, FIG. 3(b) is a bottom view of the camera head adaptor as viewed from below, and FIG. 3(c) is a perspective view of the camera head adaptor as viewed from the bottom side.
FIG. 4(a) is a perspective view illustrating a state in which the camera head adaptor, the holder, and an attachment member of the arm portion are joined to each other and FIG. 4(b) is a perspective view illustrating a state in which the camera head adaptor, the holder, and the attachment member of the arm portion are separated from each other.
FIG. 5(a) is a perspective view of the holder as viewed from the rear upper side and FIG. 5(b) is a perspective view of the holder as viewed from the front lower side.
FIG. 6(a) is a plan view of the holder as viewed from above, FIG. 6(b) is a rear view of the holder as viewed from the rear side, and FIG. 6(c) is a front view of the holder as viewed from the front side.
FIG. 7 is a diagram in which a cross-sectional view of the holder as viewed along the C-C' line of FIG. 6 and a diagram virtually illustrating a right-side wall portion of the connecting portion of the camera head adaptor to be inserted into the holder are combined and illustrated.
FIG. 8(a) is a diagram illustrating a state in which the connecting portion is displaced downward from the state of FIG. 7 and inserted in the holder and FIG. 8(b) is a diagram illustrating a state in which the connecting portion is displaced frontward from the state of FIG. 8(a).

### [Embodiments for Carrying out the Invention]

Hereinafter, the camera head adaptor according to one or more embodiments of the present invention will be described in detail with reference to the drawings.

In the following description, an observation optical axis AX1 of an endoscope 100 and a central axis AX2 along the longitudinal direction of a camera head adaptor 20 will be set, the front-rear direction along the central axis AX2 will be referred to as a D11-D12 direction, and the up-down direction and the right-left direction (width direction) which are orthogonal to the front-rear direction and orthogonal to each other will be referred to as a D21-D22 direction and D31-D32 direction, respectively. The central axis AX2 coincides approximately with the observation optical axis AX1 when the endoscope 100 is attached to the camera head adaptor 20. In each figure, D11, D12, D21, D22, D31, and D32 may be referred to as a front side, a rear side, an upper side, a lower side, a left side, and a right side, respectively.

In the present embodiment, an example of holding the endoscope 100 used for endoscopic surgery will be described for a form in which a holder 60 can be attached to a robot for surgical assistance as a support structure. Examples of the support structure of the present embodiment include a robot whose arm portion can be bent, for example, by pneumatic drive, but the camera head adaptor 20 of the present embodiment can also be applied to a robot of a different operating principle, a robot whose arm portion is not bent for drive, and a structure other than a robot.

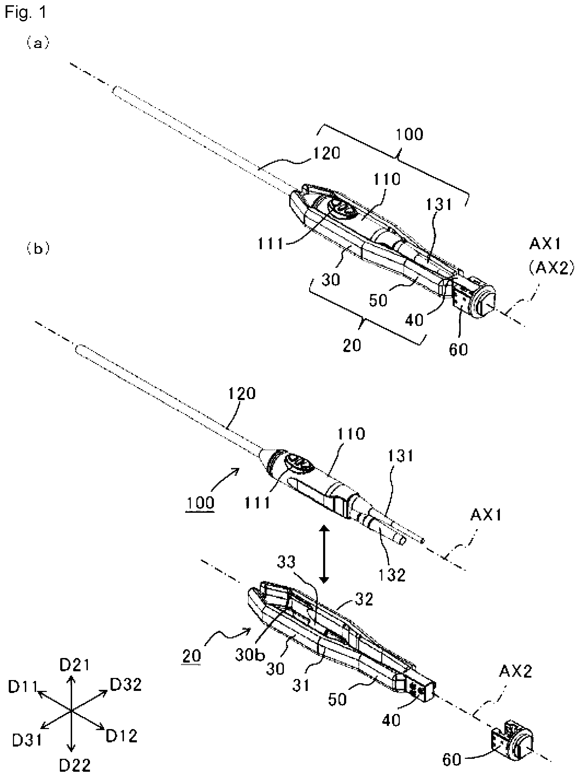

As illustrated in FIG. 1(a), the camera head adaptor 20 holds the endoscope 100 in a detachable manner. The camera head adaptor 20 is detachably attached to an attachment member 11 of the arm portion of the robot via the holder 60.

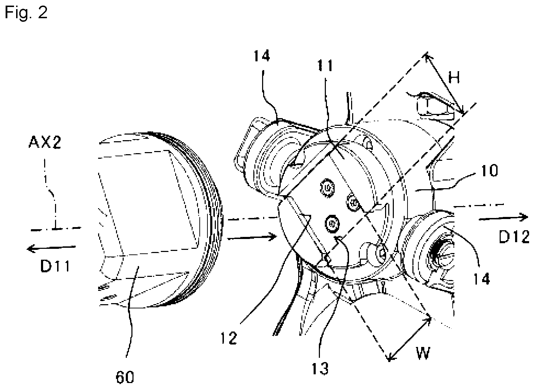

As illustrated in FIG. 2, the arm portion of the robot includes a tip holding unit 10 and a gimbal mechanism unit 14. The holder 60 is attached to the tip of the tip holding unit 10. As illustrated in FIG. 2, the tip holding unit 10 is attached to the gimbal mechanism unit 14 and has an approximately circular shape in a plan view as viewed along the central axis AX2 of the camera head adaptor 20.

The tip holding unit 10 holds the holder 60 in a detachable manner via the attachment member 11 provided at the tip portion and thereby holds the camera head adaptor 20 attached to the holder 60. The attachment member 11 is disposed concentrically with the tip holding unit 10 in a plan view along the central axis AX2 and has a circular shape having a smaller diameter than that of the tip holding unit 10.

The gimbal mechanism unit 14 supports the camera head adaptor 20 so that the camera head adaptor 20 is rotatable around the central axis AX2 along the observation optical axis AX1 of the endoscope 100 and also rotatable around a direction intersecting the central axis AX2, preferably a direction extending orthogonally to the central axis AX2.

### <Endoscope>

As illustrated in FIG. 1(b), the endoscope 100 includes a camera head 110, an insertion portion 120, an electrical cable 131, and an optical cable 132 and has an operation unit 111 on the upper surface.

The camera head 110 includes an imaging element (not illustrated) for capturing the image of an object to be observed/imaged and an optical system (not illustrated) for forming an image on the imaging element and has the observation optical axis AX1 corresponding to the optical axis of the imaging element. The camera head 110 has a change in the outer shape when viewed along the direction in which the observation optical axis AX1 extends. Specifically, the camera head 110 has a diameter that decreases in a front-side portion (D11 side portion) in the observation optical axis AX1 toward the front and decreases in a rear-side portion (D12 side portion) in the observation optical axis AX1 toward the rear, and the intermediate portion between them is in a cylindrical shape having approximately the same diameter.

The housing of the camera head 110 is composed of a rigid material in order to protect the built-in imaging element and optical system and to reliably maintain the arrangement relationship between them. On the other hand, the tubular material constituting the outer circumference of the insertion portion 120 is composed of a flexible material that can be bent and deformed when inserted into the patient's body. In addition, the tubular materials constituting the outer circumferences of the electrical cable 131 and the optical cable 132 are composed of flexible materials that allow the deformation in accordance with the routing arrangement. The housing of the camera head 110, the tubular material of the insertion portion 120, the tubular material of the electrical cable 131, and the tubular material of the optical cable 132 are composed, for example, of resin materials as the materials having the above-described properties.

The user can operate the operation unit 111 thereby to perform the start/completion of the imaging, the adjustment of the imaging conditions by operating the built-in optical system, the adjustment of the illumination intensity, etc.

The insertion portion 120 is provided on one end portion side of the camera head 110 in the direction along the observation optical axis AX1, and the electrical cable 131 and the optical cable 132 are provided on the other side in the direction along the observation optical axis AX1. Thus, the insertion portion 120, the camera head 110, the electrical cable 131, and the optical cable 132 are arranged in this order from the object side along the observation optical axis AX1.

The electrical cable 131 and the optical cable 132 are smaller in size than the camera head 110 in the width direction (D31-D32 direction) and are arranged side by side in the up-down direction (D21-D22 direction).

The insertion portion 120 is provided so as to extend along the observation optical axis AX1 and has, in its inside, an optical fiber or the like that guides the light emitted from an external light source to the tip surface and a path that guides the light incident from the tip surface to the camera head 110. The insertion portion 120 may be provided therein with a treatment tool for treating an object, such as forceps or a path for ejecting water or air, a wire for bending and deforming the insertion portion 120, etc.

The electrical cable 131 is configured such that a supply line for supplying the electric power to the camera head 110 and a transmission line for transmitting the imaging data from the imaging element to an external image processing unit or display unit are inserted in the electrical cable 131. The optical cable 132 has a path such as an optical fiber for guiding the emitted light from the external light source to the insertion portion 120 side.

### <Camera Head Adaptor>

As illustrated in FIG. 1(b), the camera head adaptor 20 is formed so as to extend along the direction of the central axis AX2 parallel to the observation optical axis AX1 of the endoscope 100 and includes a holding portion 30 located on the front side, a connecting portion 40 located on the rear side, and a coupling portion 50 that couples the holding portion 30 and the connecting portion 40 to each other.

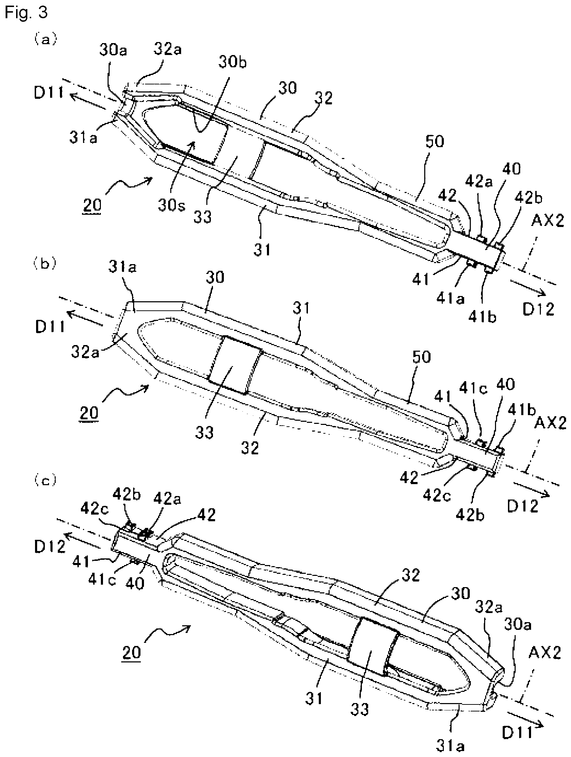

The holding portion 30 holds the camera head 110, which has a rigid housing, of the endoscope 100 in a detachable manner. As illustrated in FIGS. 3(a), 3(b), and 3(c), the holding portion 30 extends along the direction of the central axis AX2 and includes two side wall portions 31 and 32 facing each other across the central axis AX2 and a bottom wall portion 33 that connects the two side wall portions 31 and 32 to each other on the back side in the insertion direction of the camera head 110 into the holding portion 30. The two side wall portions 31 and 32 have a facing distance that decreases in the front-side (D11 side) portion in the direction along the central axis AX2 toward the front and decreases in the rear-side (D12 side) portion toward the rear so as to correspond to the shape of the housing of the camera head 110, and the intermediate portion between them has approximately the same facing distance. Thus, the holding portion 30 has an inner surface shape corresponding to the outer shape of the camera head 110.

Front tip portions 31a and 32a of the two side wall portions 31 and 32 are connected to each other, and a groove portion 30a along the central axis AX2 is provided between the front tip portions 31a and 32a. The upper side of the groove portion 30a is open. That is, the groove portion 30a is formed in the front-side portion of the holding portion 30. When the camera head 110 is disposed in the holding portion 30 of the camera head adaptor 20, the insertion portion 120 extends to the outside through the groove portion 30a; therefore, the insertion portion 120 can be extended without bending, and the camera head 110 can be held while suppressing the stress applied to the insertion portion 120. Moreover, the insertion portion 120 is supported by being in contact with the groove portion 30a.

As substitute for the groove portion 30a, a hole portion may be provided so as to pass through the connection portion between the front tip portions 31a and 32a of the two side wall portions 31 and 32 along the direction of the central axis AX2.

The camera head adaptor 20 is manufactured, for example, by integral molding of a resin. In the camera head adaptor 20, the two side wall portions 31 and 32 have an opening portion 30b with which the camera head 110 can be inserted in a space 30s between the side wall portions 31 and 32 (see FIG. 3(a)). The opening portion 30b is positioned and sized so that the operation unit 111 is exposed when the camera head 110 is inserted and arranged in the above space 30s.

The camera head adaptor 20 has flexibility so as to be capable of reliably holding the camera head 110 in the above space in a normal surgical operation and allowing the camera head 110 to be inserted into the above space 30s by elastic deformation of the two side wall portions 31 and 32. In the holding portion 30, the camera head 110 inserted in the space 30s between the two side wall portions 31 and 32 is supported by the two side wall portions 31 and 32 at the two sides of the camera head 110 facing each other and also supported by the bottom wall portion 33 at the bottom portion of the camera head 110. When the two side wall portions 31 and 32 have shapes in which the curvature, bend, or thickness is adjusted so that the upper end portions (ridge portions on the upper side (D21 side)) of the two side wall portions 31 and 32 are closer to each other than the lower end portions are, and the two side wall portions 31 and 32 are in contact with or close to a part of the upper surface of the camera head 110 accommodated therein, this is preferred because the camera head 110 can be reliably held.

The side wall portions may be composed of a plurality of three or more wall portions, provided that the camera head 110 can be reliably held.

By configuring the holding portion 30 in this way, the camera head 110 can be reliably held in the space 30s, and the holding portion 30 serves as a release structure that is configured such that the holding of the camera head 110 can be released by elastic deformation or plastic deformation of the two wall portions 31 and 32 when an external force of a predetermined value or more is applied to the endoscope 100 or the camera head adaptor 20. The above predetermined value is preliminarily set as a value sufficiently large with respect to the external force applied during the operation in a normal surgical operation or setting operation, and the camera head 110 can be released from the camera head adaptor 20 when an external force of the predetermined value or more is applied. The shapes and flexibility (elasticity) of the two side wall portions 31 and 32 of the camera head adaptor 20 are set so that such a release structure functions.

The coupling portion 50 is composed of portions in which the two side wall portions 31 and 32 extend from the holding portion 30, and the space between the two side wall portions 31 and 32 merges into the space 30s in the holding portion 30. The facing distance between the two side wall portions 31 and 32 in the width direction (D31-D32 direction) is set smaller than that of the holding portion 30. This facing distance is smaller than the camera head 110 and is large enough to allow the thickest portions of the electrical cable 131 and the optical cable 132 to be inserted. The coupling portion 50 is not provided with a bottom portion and has a shape passing through in the up-down direction. The camera head 110 cannot be inserted into the coupling portion 50 due to such a shape, and therefore the user can easily insert the camera head 110 into the holding portion 30, which is the correct insertion position, simply by adjusting the camera head 110 back and forth. Moreover, it is possible to prevent disconnection and other problems because the connection portion in which the electrical cable 131 and the optical cable 132 are connected to the camera head 110 is inserted in the coupling portion 50 without bending, so that the connection portions between the camera head 110 and the electrical cable 131 and optical cable 132 are less likely to be stressed during the operation of the endoscope or robot.

The coupling portion 50 is not limited to the above structure, provided that it can reliably couple the holding portion 30 and the connecting portion 40. For example, another configuration is also possible in which the two side wall portions 31 and 32 are not extended to the coupling portion 50, but are joined to each other on the near side of the coupling portion 50 to form a closed shape and a rod-shaped coupling portion or a plate-shaped coupling portion, for example, is joined to the holding portion having such a shape.

In the present embodiment, the two side wall portions 31 and 32 are flexible (elastic), but can be composed of a rigid material, provided that the camera head 110 can be inserted and reliably held. In this case, it is preferred to give the coupling portion 50 flexibility or elasticity because the insertability of the camera head 110 can be ensured.

As illustrated in FIGS. 3(a) to 3(c), the connecting portion 40 is in a plate shape extending rearward from the rear end of the coupling portion 50 and has two side surfaces 41 and 42 facing each other.

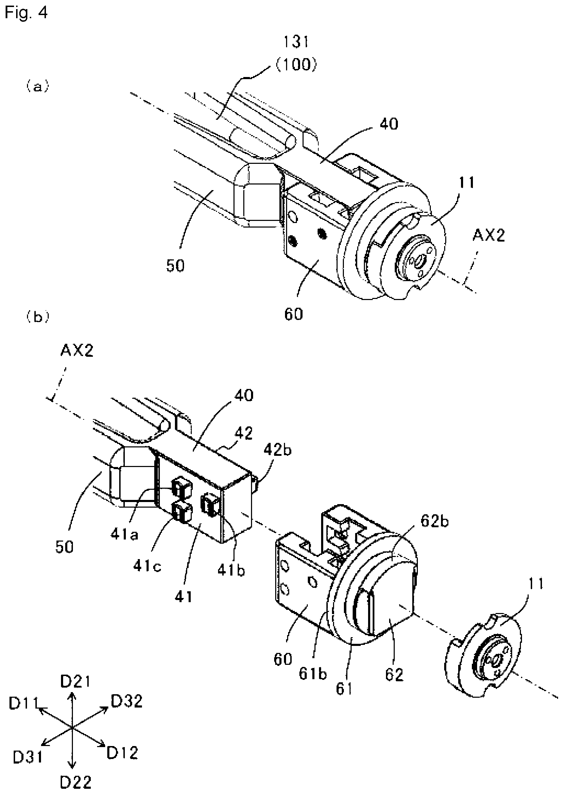

As illustrated in FIGS. 3(a) to 3(c) or FIG. 4(b), the left side surface 41 of the connecting portion 40 is provided with three engaging protrusions 41a, 41b, and 41c protruding leftward as engaging portions. The two engaging protrusions 41a and 41b on the upper side are provided at the same positions in the up-down direction (D21-D22 direction), and the two engaging protrusions 41a and 41c on the front side are provided at the same positions in the front-rear direction (D11-D12 direction). The three engaging protrusions 41a, 41b, and 41c have the same shape as each other.

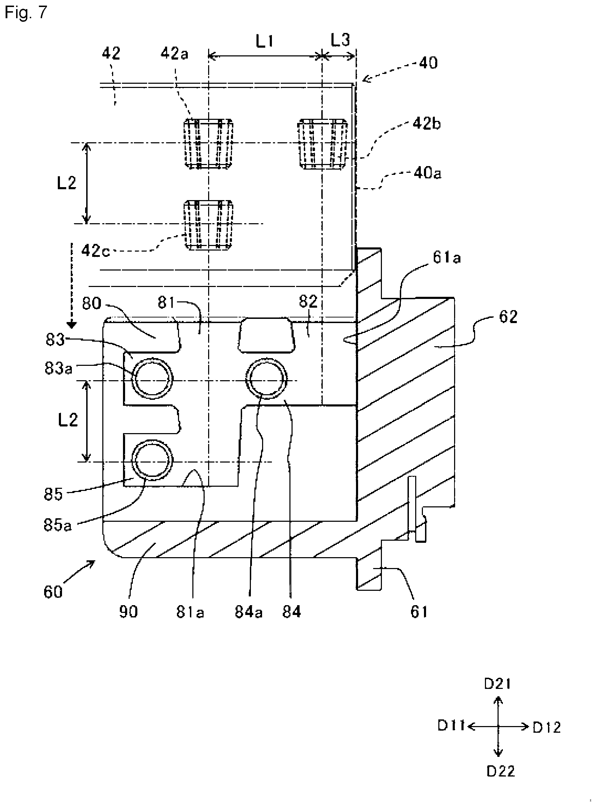

As illustrated in FIGS. 3(a) to 3(c) or FIG. 7, the right side surface 42 is provided with three engaging protrusions 42a, 42b, and 42c protruding rightward as engaging portions. As illustrated in FIG. 7, the two engaging protrusions 42a and 42b on the upper side are provided at the same positions in the up-down direction, and the center positions are arranged at a distance L1 in the front-rear direction. The two engaging protrusions 42a and 42c on the front side are provided at the same positions in the front-rear direction, and the center positions are arranged at a distance L2 in the up-down direction. The distance between the center position of the engaging protrusion 42b on the rear side and a rear end surface 40a of the connecting portion 40 is set to a distance L3. The three engaging protrusions 42a, 42b, and 42c have the same shape as each other and also have the same shapes as those of the three engaging protrusions 41a, 41b, and 41c on the left side surface 41.

These three engaging protrusions 42a, 42b, and 42c are arranged at positions corresponding to those of the three engaging protrusions 41a, 41b, and 41c on the left side surface 41 with respect to the surface extending in the up-down direction and including the central axis AX2.

### <Holder>

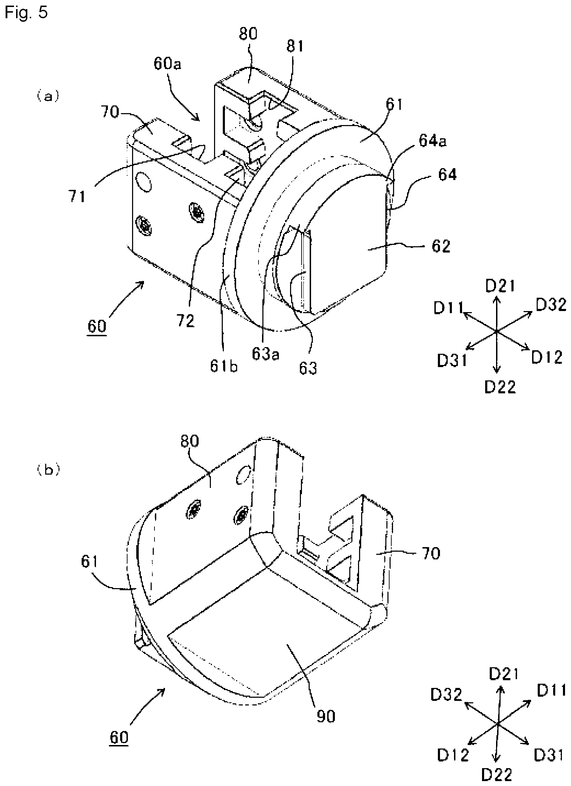

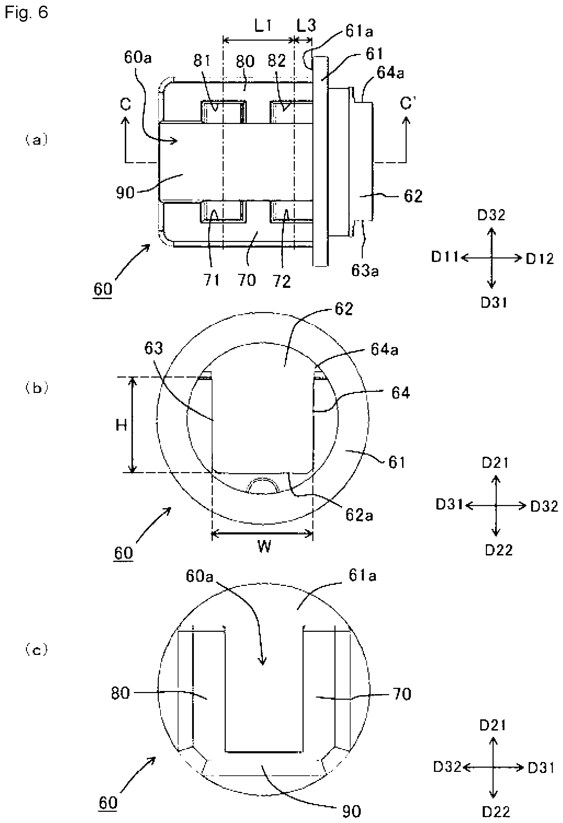

As illustrated in FIGS. 5(a) and 5(b) and FIGS. 6(a) to 6(c), the holder 60 includes a base material portion 61 that is provided at the rear portion and two side wall portions 70 and 80 and a bottom wall portion 90 that are provided on a front surface 61a of the base material portion 61. The base material portion 61, the two side wall portions 70 and 80, and the bottom wall portion 90 are manufactured, for example, by integral molding of a rigid resin material.

The base material portion 61 has a planar shape that spreads approximately like a circular disk when viewed along the front-rear direction (D11-D12 direction), and the rear portion of the base material portion 61 is provided with an attaching portion 62 that protrudes rearward and is joined to the arm portion of a robot in a detachable manner.

The holder 60 may be configured to be fixed to the arm portion of a robot, or the attachment member of the arm portion may have the same configuration as the holder 60.

The attaching portion 62 is provided within a diameter range smaller than the diameter of the planar shape of the base material portion 61 and has a predetermined width W (see FIG. 6(b)) in the right-left direction (D31-D32 direction) and a shape extending along the up-down direction (D21-D22 direction). In the attaching portion 62, overhanging portions 63a and 64a extending outward in the right-left direction are provided on the upper portions of two side surfaces 63 and 64 facing each other in the right-left direction, respectively. In the state illustrated in FIG. 6(b), the attaching portion 62 has a predetermined height H from a bottom portion 62a to the overhanging portions 63a and 64a.

The two side wall portions 70 and 80 of the holder 60 are provided so as to extend from the front surface 61a of the base material portion 61 to the front side (D11 side) and face each other in the right-left direction (D31-D32 direction). The bottom wall portion 90 extends from the front surface 61a of the base material portion 61 to the front side and is connected to the lower portions of the two side wall portions 70 and 80. This allows a space 60a to be formed in front of the base material portion 61. The space 60a is surrounded by the two side wall portions 70 and 80 and the bottom wall portion 90, and the upper side of the space 60a is open. The camera head adopter holding portion is thus configured, which holds the camera head adaptor 20 in a detachable manner.

As illustrated in FIG. 6(a), the upper inner surface of the left-side first side wall portion 70 is provided with two insertion recessed portions 71 and 72 that are recessed toward the outside (left side (D31 side)). These insertion recessed portions 71 and 72 are provided side by side so that the center positions are arranged at a predetermined distance L1 in the front-rear direction.

The upper inner surface of the right-side second side wall portion 80 of the holder 60 is provided with two insertion recessed portions 81 and 82 that are recessed toward the outside (right side (D32 side)) so as to face the two insertion recessed portions 71 and 72 of the left-side first side wall portion 70, respectively. These insertion recessed portions 81 and 82 are provided side by side so that the center positions are arranged at a predetermined distance L1 in the front-rear direction. In the rear-side insertion recessed portion 82, its center position is separated from the front surface 61a of the base material portion 61 by a distance L3 in the front-rear direction.

As illustrated in FIG. 7, the front-side insertion recessed portion 81 of the right-side second side wall portion 80 is provided in a linked manner with a first fixing recessed portion 83 extending frontward at a position at which a predetermined distance is extended downward, and the rear-side insertion recessed portion 82 is provided in a linked manner with a second fixing recessed portion 84 extending frontward at a position at which the same distance as that of the front-side insertion recessed portion 81 is extended downward. The second fixing recessed portion 84 is provided in a linked manner with the insertion recessed portion 81 in the front-rear direction and is provided at the same height position as the first fixing recessed portion 83 in the up-down direction.

The front-side insertion recessed portion 81 extends further downward from the linked portion with the first fixing recessed portion 83 and is provided in a linked manner with a third fixing recessed portion 85 extending frontward at a position at which a distance L2 is extended. Here, the three fixing recessed portions 83, 84, and 85 are arranged so that the relative positional relationships of the three fixing recessed portions 83, 84, and 85 in the up-down and front-rear directions correspond to the relative positional relationships of the three engaging protrusions 42a, 42b, and 42c of the connecting portion 40 in the up-down and front-rear directions. The three fixing recessed portions 83, 84, and 85 have inner surface shapes corresponding to the outer shapes of the three engaging protrusions 42a, 42b, and 42c, respectively. When the three engaging protrusions 42a, 42b, and 42c are inserted, therefore, they are press-fitted by applying a predetermined force, and after the press-fitting, they are fixed to such an extent that they are not detached even if an external force due to an operation such as a normal surgical operation is applied.

Here, although not illustrated, the insertion recessed portions 71 and 72 of the left-side first side wall portion 70 are also provided in a linked manner with a first fixing recessed portion, a second fixing recessed portion, and a third fixing recessed portion as in the insertion recessed portions 81 and 82 of the right-side second side wall portion 80.

The joint between the camera head adaptor 20 and the holder 60 will be described by focusing on the joint between the right side surface 42 of the connecting portion 40 and the right-side second side wall portion 80 of the holder 60. The left side surface 41 of the connecting portion 40 and the left-side first side wall portion 70 of the holder 60 are also joined in the same manner.

First, as illustrated in FIG. 7, the camera head adaptor 20 is located above the holder 60, and the lower portion of the rear end surface 40a of the connecting portion 40 is brought into contact with the upper portion of the front surface 61a of the base material portion 61. This allows the engaging protrusions 42c and 42b of the connecting portion 40 to be located above the insertion recessed portions 81 and 82, respectively. At this time, although not illustrated, the engaging protrusions 41c and 41b of the connecting portion 40 are located above the insertion recessed portions 71 and 72 of the holder 60, respectively.

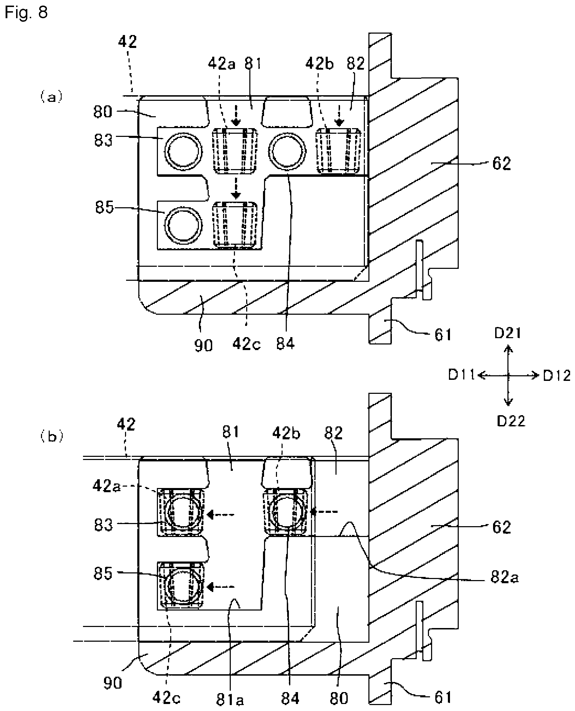

Then, the camera head adaptor 20 is displaced downward or the holder 60 is displaced upward, and the engaging protrusions 42c and 42b of the connecting portion 40 are thereby inserted into the insertion recessed portions 81 and 82, respectively (FIG. 8(a)). At this time, the engaging protrusions 41c and 41b of the connecting portion 40 are also inserted into the insertion recessed portions 71 and 72, respectively.

Subsequently, taking the right side surface 42 as an example, as illustrated in FIG. 8(a), the engaging protrusion 42c is brought into contact with a bottom portion 81a of the insertion recessed portion 81 while the engaging protrusion 42b is brought into contact with a bottom portion 82a of the insertion recessed portion 82, and the insertion into the second side wall portion 80 is thereby restricted in the up-down direction. Then, by moving the camera head adaptor 20 frontward or the holder 60 rearward from the state of FIG. 8(a), the engaging protrusion 42a is press-fitted into the first fixing recessed portion 83, the engaging protrusion 42b is press-fitted into the second fixing recessed portion 84, and the engaging protrusion 42c is press-fitted into the third fixing recessed portion 85 (FIG. 8(b)). Through this operation, the connecting portion 40 and the holder 60 are joined, and the camera head adaptor 20 and the holder 60 are fixed to each other.

Here, as illustrated in FIG. 7, in the three fixing recessed portions 83, 84, and 85, it is preferred to provide respective recessed portions or through holes that are recessed in the second side wall portion 80 along the right-left direction (D31-D32 direction), and dispose, in the recessed portions or through holes, elastic members 83a, 84a, and 85a that can be elastically extended toward the inside of the holder 60 along the right-left direction. Through this configuration, when the engaging protrusions 42a, 42b, and 42c are press-fitted into the three fixing recessed portions 83, 84, and 85, respectively, elastic forces can be applied along the right-left direction, and the camera head adaptor 20 and the holder 60 can therefore be more firmly fixed to each other.

When the camera head adaptor 20 is moved rearward or the holder 60 is moved frontward from the state in which the camera head adaptor 20 and the holder 60 are fixed to each other as above, the engaging protrusion 42a, the engaging protrusion 42b, and the engaging protrusion 42c are extracted from the first fixing recessed portion 83, the second fixing recessed portion 84, and the third fixing recessed portion 85, respectively. Then, by pulling up the connecting portion 40 upward to pull out the engaging protrusion 42a, the engaging protrusion 42b, and the engaging protrusion 42c from the insertion recessed portions 81 and 82, it is possible to release the camera head adaptor 20 from the holder 60. Thus, the above steps allow the holder 60 to hold the camera head adaptor 20 in a detachable manner.

The attaching portion 62 of the holder 60 has a shape corresponding to a holding groove portion 12 provided in the attachment member 11 of the tip holding unit 10 of the robot's arm portion. Specifically, as illustrated in FIG. 2, the holding groove portion 12 is a rectangular groove having a width W and a height H in a plan view, and one end in the direction of the height H is an opening portion 13. The holder 60 can therefore be joined to the tip holding unit 10 in a detachable manner by press-fitting the attaching portion 62 from the opening portion 13 of the holding groove portion 12.

Here, an outer peripheral surface 61b of the base material portion 61 of the holder 60 or an outer peripheral surface 62b of the attaching portion 62 may be used, for example, as a drape holding portion. When the tip portion of a drape is attached to any or each of the outer peripheral surfaces 61b and 62b and the drape is made to cover the arm portion or further the robot as a whole, the robot can be reliably kept in a clean state, and the holder 60 and the camera head adaptor 20 contaminated by the surgical operation or the like can be easily separated from the robot.

### [Industrial Applicability]

As described above, the camera head adaptor according to the present invention is useful in that it allows an endoscope to be detachably attached with a simple operation and it can prevent stress on electrical wirings, optical cables, etc. in the insertion portion due to the camera head being held.

### [Description of Reference Numerals]

- 10: Tip holding unit
- 11: Attachment member
- 12: Holding groove portion
- 13: Opening portion
- 14: Gimbal mechanism unit
- 20: Camera head adaptor
- 30: Holding portion
- 30a: Groove portion
- 30b: Opening portion
- 30s: Space
- 31, 32: Side wall portion
- 31a, 32a: Front tip portion
- 33: Bottom wall portion
- 40: Connecting portion
- 40a: Rear end surface
- 41: Left side surface
- 41a, 41b, 41c: Engaging protrusion
- 42: Right side surface
- 42a, 42b, 42c: Engaging protrusion
- 50: Coupling portion
- 60: Holder
- 60a: Space
- 61: Base material portion
- 61a: Front surface
- 61b: Outer peripheral surface
- 62: Attaching portion
- 62a: Bottom portion
- 62b: Outer peripheral surface
- 63, 64: Side surface
- 63a, 64a: Overhanging portion
- 70: First side wall portion
- 71, 72: Insertion recessed portion
- 80: Second side wall portion
- 81, 82: Insertion recessed portion
- 81a, 82a: Bottom portion
- 83, 84, 85: Fixing recessed portion
- 83a, 84a, 85a: Elastic member
- 90: Bottom wall portion
- 100: Endoscope
- 110: Camera head
- 111: Operation unit
- 120: Insertion portion
- 131: Electrical cable
- 132: Optical cable
- AX1: Observation optical axis
- AX2: Central axis
- H: Height
- L1, L2, L3: Distance
- W: Width

## Claims

1. A camera head adaptor (20) for holding an endoscope (100) having a camera head (110), comprising:
a holding portion (30) that holds the camera head (110) of the endoscope (100) in a detachable manner;
a connecting portion (40) that extends along an observation optical axis (AX1) of the camera head (110) and can be attached to a support structure; and
a coupling portion (50) that couples the holding portion (30) and the connecting portion (40),
wherein
the holding portion (30) is composed of an elastically deformable material and comprises a plurality of wall portions that elastically hold the camera head (110) inserted in the holding portion (30) while being in contact with the camera head (110),
the plurality of wall portions of the holding portion (30) includes two side wall portions (31, 32) that extend along a direction of a central axis (AX2) of the holding portion (30) and face each other and the camera head (110) is supported by the two side wall portions (31, 32),
and **characterised in that** the coupling portion (50) is composed of portions in which the two side wall portions (31, 32) extend from the holding portion 30.

2. The camera head adaptor according to claim 1, wherein the holding portion (30) has a release structure that can release holding of the camera head (110) when an external force of a predetermined value or more is applied to the endoscope (100) or the camera head adaptor (20).

3. The camera head adaptor according to claim 2, wherein
the holding portion (30) is composed of an elastically deformable material and comprises said plurality of wall portions that elastically hold the camera head (110) inserted in the holding portion (30) while being in contact with the camera head (110), and
the release structure is configured such that the holding of the camera head (110) is released by elastic deformation or plastic deformation of the plurality of wall portions when the external force of the predetermined value or more is applied to the endoscope (100) or the camera head adaptor (20).

4. The camera head adaptor according to claim 1, wherein the connecting portion (40) comprises an engaging portion that enables detachable attachment to the support structure.

5. The camera head adaptor according to claim 4, wherein the support structure is a robot comprising a bendable arm portion, and the connecting portion (40) is detachably attached to the arm portion.

6. The camera head adaptor according to any one of claims 1 to 4, wherein
the endoscope (100) is configured such that an insertion portion (120), an electrical cable (131), and an optical cable (132) extend from the camera head (110),
the holding portion (30) holds only the camera head (110) of the endoscope (100), and
the electrical cable (131) and the optical cable (132) extend from the coupling portion (50) to outside.

7. The camera head adaptor according to claim 6, wherein the camera head (110) has a change in an outer shape when viewed along a direction in which the observation optical axis (AX1) extends, and the holding portion (30) has an inner surface shape corresponding to the outer shape of the camera head (110).

8. The camera head adaptor according to one of the claims 1 to 7, wherein the two side wall portions (31, 32) have a facing distance that decreases in a front-side portion in the direction of the central axis (AX2) toward a front and decreases in a rear-side portion toward a rear.

9. The camera head adaptor according to one of the claims 1 to 7, wherein the plurality of wall portions of the holding portion (30) further includes a bottom wall portion (90) that connects the two side wall portions to each other on a back side in an insertion direction of the camera head (110), and the camera head (110) is supported by the two side wall portions (31, 32) and the bottom wall portion (90).

10. The camera head adaptor according to any one of claims 1 to 9, wherein a front-side portion of the holding portion (30) is provided with a groove portion (30a) that allows the insertion portion (120) extending from the camera head (110) held by the holding portion (30) to extend to outside without bending.

11. The camera head adaptor according to one of the claims 1 to 7, wherein an opening portion (30b) that allows the camera head (110) to be inserted is formed between the two side wall portions (31, 32), and when the camera head (110) is inserted in the holding portion (30), an operation unit of the camera head (110) is exposed from the opening portion (30b).

12. The camera head adaptor according to claim 1, wherein the coupling portion (50) has a space that merges into a space accommodating the camera head (110) in the holding portion (30), and the electrical cable (131) and the optical cable (132) are connected to the camera head (110) without bending.

13. The camera head adaptor according to one of the claims 1 to 12, wherein the connecting portion (40) is placed in the optical axis (AX1).

## Patentansprüche

1. Kamerakopfadapter (20) zum Halten eines Endoskops (100) mit einem Kamerakopf (110), enthaltend:
einen Halteabschnitt (30), der den Kamerakopf (110) des Endoskops (100) in einer lösbaren Weise hält;
einen Verbindungsabschnitt (40), der sich entlang einer optischen Beobachtungsachse (AX1) des Kamerakopfes (110) erstreckt und an einer Stützstruktur befestigt sein kann; und
einen Kopplungsabschnitt (50), der den Halteabschnitt (30) und den Verbindungsabschnitt (40) koppelt,
wobei der Halteabschnitt (30) aus einem elastisch verformbaren Material zusammengesetzt ist und eine Vielzahl von Wandabschnitten enthält, die den in den Halteabschnitt (30) eingesetzten Kamerakopf (110) elastisch halten, während er mit dem Kamerakopf (110) in Kontakt steht,
die Vielzahl von Wandabschnitten des Halteabschnitts (30) zwei Seitenwandabschnitte (31, 32) einschließt, die sich entlang einer Richtung einer zentralen Achse (AX2) des Halteabschnitts (30) erstrecken und einander zugewandt sind und der Kamerakopf (110) von den beiden Seitenwandabschnitten (31, 32) gestützt wird und
und **dadurch gekennzeichnet, dass** der Kopplungsabschnitt (50) aus Abschnitten zusammengesetzt ist, in denen sich die beiden Seitenwandabschnitte (31, 32) von dem Halteabschnitt 30 erstrecken.

2. Kamerakopfadapter nach Anspruch 1, wobei der Halteabschnitt (30) eine Freigabestruktur aufweist, die das Halten des Kamerakopfes (110) freigeben kann, wenn eine äußere Kraft eines vorbestimmten Wertes oder mehr auf das Endoskop (100) oder den Kamerakopfadapter (20) ausgeübt wird.

3. Kamerakopfadapter nach Anspruch 2, wobei
der Halteabschnitt (30) aus einem elastisch verformbaren Material zusammengesetzt ist und eine Vielzahl von Wandabschnitten enthält, die den in den Halteabschnitt (30) eingesetzten Kamerakopf (110) elastisch halten, während er mit dem Kamerakopf (110) in Kontakt steht, und
die Freigabestruktur konfiguriert ist, so dass das Halten des Kamerakopfes (110) durch elastische Verformung oder plastische Verformung der Vielzahl von Wandabschnitte freigegeben wird, wenn die äußere Kraft des vorgegebenen Wertes oder mehr auf das Endoskop (100) oder den Kamerakopfadapter (20) ausgeübt wird.

4. Kamerakopfadapter nach Anspruch 1, wobei der Verbindungsabschnitt (40) einen Eingriffsabschnitt aufweist, der eine lösbare Befestigung an der Stützstruktur ermöglicht.

5. Kamerakopfadapter nach Anspruch 4, wobei die Stützstruktur ein Roboter ist, der einen biegsamen Armabschnitt enthält, und der Verbindungsabschnitt (40) lösbar an dem Armabschnitt befestigt ist.

6. Kamerakopfadapter nach einem der Ansprüche 1 bis 4, wobei
das Endoskop (100) konfiguriert ist, so dass sich ein Einführabschnitt (120), ein elektrisches Kabel (131) und ein optisches Kabel (132) von dem Kamerakopf (110) erstrecken,
der Halteabschnitt (30) nur den Kamerakopf (110) des Endoskops (100) hält und
das elektrische Kabel (131) und das optische Kabel (132) sich von dem Kopplungsabschnitt (50) nach außen erstrecken.

7. Kamerakopfadapter nach Anspruch 6, wobei der Kamerakopf (110) eine Änderung in einer äußeren Form aufweist, wenn entlang einer Richtung betrachtet, in der sich die optische Beobachtungsachse (AX1) erstreckt, und der Halteabschnitt (30) eine Innenflächenform aufweist, die der äußeren Form des Kamerakopfes (110) entspricht.

8. Kamerakopfadapter nach einem der Ansprüche 1 bis 7, wobei die zwei Seitenwandabschnitte (31, 32) einen zugewandten Abstand aufweisen, der in einem vorderseitigen Abschnitt in die Richtung der zentralen Achse (AX2) in Richtung einer Vorderseite abnimmt und in einem rückwärtigen Abschnitt in Richtung einer Rückseite abnimmt.

9. Kamerakopfadapter nach einem der Ansprüche 1 bis 7, wobei die Vielzahl von Wandabschnitten des Halteabschnitts (30) ferner einen Bodenwandabschnitt (90) einschließen, der die zwei Seitenwandabschnitte auf einer Rückseite in einer Einführrichtung des Kamerakopfes (110) miteinander verbindet, und der Kamerakopf (110) durch die beiden Seitenwandabschnitte (31, 32) und den unteren Wandabschnitt (90) gestützt wird.

10. Kamerakopfadapter nach einem der Ansprüche 1 bis 9, wobei ein vorderseitiger Abschnitt des Halteabschnitts (30) mit einem Nutabschnitt (30a) bereitgestellt ist, der erlaubt, dass sich der Einführabschnitt (120), der sich von dem von dem Halteabschnitt (30) gehaltenen Kamerakopf (110) erstreckt, nach außen erstrecken kann, ohne sich zu verbiegen.

11. Kamerakopfadapter nach einem der Ansprüche 1 bis 7, wobei zwischen den zwei Seitenwandabschnitten (31, 32) ein Öffnungsabschnitt (30b) gebildet ist, der dem Kamerakopf (110) erlaubt, eingeführt zu werden, und wenn der Kamerakopf (110) in den Halteabschnitt (30) eingeführt ist, eine Betätigungseinheit des Kamerakopfes (110) von dem Öffnungsabschnitt (30b) freigelegt ist.

12. Kamerakopfadapter nach Anspruch 1, wobei der Kopplungsabschnitt (50) einen Raum aufweist, der in einen den Kamerakopf (110) unterbringenden Raum in dem Halteabschnitt (30) übergeht, und das elektrische Kabel (131) und das optische Kabel (132) mit dem Kamerakopf (110) verbunden sind, ohne sich zu biegen.

13. Kamerakopfadapter nach einem der Ansprüche 1 bis 12, wobei der Verbindungsabschnitt (40) in der optischen Achse (AX1) platziert ist.

## Revendications

1. Un adaptateur de tête de caméra (20) pour maintenir un endoscope (100) ayant une tête de caméra (110), comprenant :
une partie de maintien (30) qui maintient la tête de caméra (110) de l'endoscope (100) d'une manière détachable;
une partie de connexion (40) qui s'étend le long d'un axe optique d'observation (AX1) de la tête de caméra (110) et peut être fixée à une structure de support; et
une partie d'accouplement (50) qui couple la partie de maintien (30) et la partie de connexion (40),
dans lequel la partie de maintien (30) est composée d'un matériau déformable élastiquement et comprend une pluralité de parties de paroi qui maintiennent élastiquement la tête de caméra (110) insérée dans la partie de maintien (30) tout en étant en contact avec la tête de caméra (110),
la pluralité des parties de paroi de la partie de maintien (30) comprend deux parties de paroi latérale (31, 32) qui s'étendent le long d'un axe central (AX2) de la partie de maintien (30) et se font face, et la tête de caméra (110) est soutenue par les deux parties de paroi latérale (31, 32),
et **caractérisé en ce que** la partie d'accouplement (50) est composée de parties dans lesquelles les deux parties de paroi latérale (31, 32) s'étendent de la partie de maintien 30.

2. L'adaptateur de tête de caméra selon la revendication 1, dans lequel la partie de maintien (30) présente une structure de libération qui permet de libérer le maintien de la tête de caméra (110) lorsqu'une force externe d'une valeur prédéterminée ou supérieure est appliquée à l'endoscope (100) ou à l'adaptateur de tête de caméra (20).

3. L'adaptateur de tête de caméra selon la revendication 2, dans lequel
la partie de maintien (30) est composée d'un matériau déformable élastiquement et comprend ladite pluralité de parties de paroi qui maintiennent élastiquement la tête de caméra (110) insérée dans la partie de maintien (30) tout en étant en contact avec la tête de caméra (110), et
la structure de libération est configurée de telle sorte que le maintien de la tête de caméra (110) soit libéré par déformation élastique ou déformation plastique de la pluralité de parties de paroi lorsque la force externe de la valeur prédéterminée ou supérieure est appliquée à l'endoscope (100) ou à l'adaptateur de tête de caméra (20).

4. L'adaptateur de tête de caméra selon la revendication 1, dans lequel la partie de connexion (40) comprend une partie engageante qui permet une fixation détachable à la structure de support.

5. L'adaptateur de tête de caméra selon la revendication 4, dans lequel la structure de support est un robot comprenant une partie bras pliable, et la partie de connexion (40) est fixée de manière détachable à la partie bras.

6. L'adaptateur de tête de caméra selon l'une quelconque des revendications 1 à 4, dans lequel
l'endoscope (100) est configuré de telle sorte qu'une partie d'insertion (120), un câble électrique (131) et un câble optique (132) s'étendent de la tête de caméra (110),
la partie de maintien (30) maintient seulement la tête de caméra (110) de l'endoscope (100);
le câble électrique (131) et le câble optique (132) s'étendent de la partie d'accouplement (50) vers l'extérieur.

7. L'adaptateur de tête de caméra selon la revendication 6, dans lequel la tête de caméra (110) présente un changement de forme extérieure lorsqu'elle est vue le long d'une direction dans laquelle l'axe optique d'observation (AX1) s'étend, et la partie de maintien (30) présente une forme de surface intérieure correspondant à la forme extérieure de la tête de caméra (110).

8. L'adaptateur de tête de caméra selon l'une des revendications s 1 à 7, dans lequel les deux parties de paroi latérale (31, 32) ont une distance face à face qui diminue dans une partie frontale dans la direction de l'axe central (AX2) vers un avant et diminue dans une partie arrière vers un arrière.

9. L'adaptateur de tête de caméra selon l'une des revendications s 1 à 7, dans lequel la pluralité de parties de paroi de la partie de maintien (30) comprend en outre une partie de paroi inférieure (90) qui relie les deux parties de paroi latérale l'une à l'autre sur une face arrière dans un sens d'insertion de la tête de caméra (110), et la tête de caméra (110) est soutenue par les deux parties de paroi latérale (31, 32) et la partie inférieure de la paroi (90).

10. L'adaptateur de tête de caméra selon l'une quelconque des revendications 1 à 9, dans lequel une partie frontale de la partie de maintien (30) est équipée d'une partie rainure (30a) qui permet à la partie d'insertion (120) s'étendant de la tête de caméra (110) maintenue par la partie de maintien (30) de s'étendre vers l'extérieur sans se plier.

11. L'adaptateur de tête de caméra selon l'une des revendications 1 à 7, dans lequel une partie d'ouverture (30b) qui permet d'insérer la tête de caméra (110) est formée entre les deux parties de paroi latérale (31, 32), et lorsque la tête de caméra (110) est insérée dans la partie de maintien (30), une unité de commande de la tête de caméra (110) est exposée à partir de la partie d'ouverture (30b).

12. L'adaptateur de tête de caméra selon la revendication 1, dans lequel la partie d'accouplement (50) présente un espace qui se fond dans un espace accueillant la tête de caméra (110) dans la partie de maintien (30), et le câble électrique (131) et le câble optique (132) sont connectés à la tête de caméra (110) sans se plier.

13. L'adaptateur de tête de caméra selon l'une des revendications 1 à 12, dans lequel la partie de connexion (40) est placée dans l'axe optique (AX1).
